# EUROPEAN PATENT APPLICATION

(11) **EP 1 655 001 A1**
(43) Date of publication of application: **10.05.2006**
(21) Application number: 04026484.8
(22) Date of filing: 08.11.2004
(51) Int. Cl.: A61F 2/06

(54) **Vascular prosthesis with attachment means and connecting means**

(71) Applicant: Inderbitzi, Rolf, Dr. med., 8967 Widen (CH)
(72) Inventor: Inderbitzi, Rolf, Dr. med., 8967 Widen (CH)
(74) Representative: Heinze, Ekkehard

(57) **Abstract**

An In-Out-Lay Prosthesis (IOLP) (100), a catheter device (500), and methods are provided for replacing a diseased section of a blood vessel by using a sutureless technique. The IOLP has a tubular body member (102), a fixing end (106), and a connecting end (108). The fixing end has attachment means (110) that help in fixing the IOLP to the blood vessel. The connecting end helps in joining one IOLP to another similar IOLP. The catheter device has an inflatable end (502) that helps in pressing the attachment means into the wall of the blood vessel.

## Description

### BACKGROUND

The present invention relates to a vascular prosthesis for use in the field of vascular surgery. In particular, the present invention relates to a vascular prosthesis for replacing a diseased section of a blood vessel, while minimizing or eliminating the requirement of sutures.

A localized dilatation of a blood vessel is called an aneurysm. An aneurysm results due to the weakening of the wall of the blood vessel caused by disease processes such as atherosclerotic degeneration or infections. Aortic aneurysms are the most common form of aneurysms. They are most commonly seen in the abdominal aorta, below the level of the renal arteries. The most common cause of aneurysms in this region is the atherosclerotic degeneration of the wall of the blood vessel.

Over a period of time, there is a gradual weakening of the wall of the aneurysm, leading to an increase in the size of the aneurysm. This may eventually lead to the spontaneous rupture of the wall of the aneurysm as the weakened wall of the aneurysm finally gives way. The risk of spontaneous rupture increases when the transverse size of the aneurysm becomes more than 5 cm.

The spontaneous rupture of an abdominal aortic aneurysm leads to massive internal bleeding, which can be fatal within minutes. Other problems that may be caused by abdominal aortic aneurysms include emboli to the lower limbs and compression of adjacent structures such as abdominal viscera, arteries, veins, and nerves. Compression of adjacent structures may lead to serious complications such as renal failure due to compression of the renal arteries, and early satiety, nausea, and vomiting due to compression of the abdominal viscera.

In order to avoid the risk of spontaneous rupture and other complications associated with abdominal aortic aneurysms, elective repair of abdominal aortic aneurysms is indicated. Abdominal aortic aneurysms can be repaired by three kinds of surgical approaches-open surgical repair, laparoscopic repair, and intraluminal/endovascular repair.

In open surgical repair, the abdominal cavity is opened through a large incision in the abdominal wall. After incising the abdominal wall, the aorta can be dissected by using a trans-peritoneal or a retro-peritoneal access. After dissecting the aorta, the aorta is clamped above and below the aneurysm and the aneurysm is transected. A vascular prosthesis is then stitched in place of the dilated section of the aorta.

However, open repair of the abdominal aortic aneurysm is a major surgical procedure. The operation takes around 2-4 hours. Occlusion of the aorta for a long period during the operation may be associated with complications such as myocardial infarction and ischemia of the lower limbs and intestines. Also, during open surgery, in case the wall of the aneurysm is atherosclerotic, it is difficult to suture the vascular prosthesis to the aortic wall. This increases the time required for the procedure and further increases the risk of complications.

Laparoscopic repair of abdominal aortic aneurysm is conducted with the help of special instruments that are inserted in the abdominal cavity through small incisions in the abdominal wall. As expected, the main advantages of this procedure include a short post-operative hospital stay and decreased trauma to the patient. Another advantage of the laparoscopic procedure is the reduction in the amount of blood loss during the procedure, since incisions are small and dissection of anatomical structures is done along predetermined anatomical layers.

However, it is difficult to apply sutures during laparoscopic surgery as direct viewing and palpation of the structures being sutured is not possible. Moreover, with advances in techniques of laparoscopic surgery, smaller incisions are being used to gain access to the abdominal cavity. As the size of the incisions in the abdominal wall decreases, it becomes an increasingly difficult task to apply sutures to the abdominal aorta by using laparoscopic instruments. Also, by using laparoscopic instruments the time required to effectively suture the vascular prosthesis to the aorta increases. Further, application of sutures to an atherosclerotic aortic wall during a laparoscopic procedure is especially difficult.

Increased difficulty in suturing the vascular prosthesis to the aorta and the increased time required for the application of the sutures, both during open and laparoscopic surgery, may increase the complications of surgery such as myocardial infarction and limb ischemia. Moreover, suturing the vascular prosthesis to an atherosclerotic aortic wall is difficult and time consuming. Further, as a consequence of suturing the atherosclerotic aortic wall, broken calcified plaques and intraluminal detritus may embolize to the limbs or intestines.

In light of the above discussion, there exists a need for a vascular prosthesis for sutureless anastomosis with a blood vessel. Moreover, the vascular prosthesis should minimize the time required for attaching the vascular prosthesis to the vessel wall. Further, the vascular prosthesis should be simple to use.

### SUMMARY

An object of the invention is to provide an In-Out-Lay Prosthesis (IOLP) suitable for replacing a diseased section of a blood vessel by using a sutureless technique.

Another object of the present invention is to provide a prosthetic system for replacing a diseased section of a blood vessel by using a sutureless technique.

Yet another object of the present invention is to provide a catheter device for fixing the IOLP inside a blood vessel.

In accordance with an exemplary embodiment of the present invention, an IOLP and a catheter device are provided for use in vascular surgery. The IOLP comprises a tubular body member having a lumen, a fixing end, and a connecting end. The lumen of the tubular body member acts as a conduit for the passage of blood. Attachment means are present on the fixing end, which help in attaching the IOLP to the wall of the blood vessel. A circular latch is present on the connecting end, which helps in connecting the IOLP with another similar IOLP. The catheter device helps in attaching the fixing end of the IOLP to the wall of the blood vessel.

In accordance with an exemplary embodiment of the present invention, the catheter device comprises an inflatable end, a body, and a handgrip. The inflatable end comprises an anvil balloon. The body and the handgrip are provided with a channel for inflating the anvil balloon. The anvil balloon helps in attaching the fixing end of the IOLP to wall of the blood vessel by pressing the attachment means present on the fixing end into the wall of the blood vessel. The body and the handgrip have a passage for introducing an occluding catheter. The occluding catheter has an occluding balloon at its tip. The occluding balloon helps in occluding the lumen of the blood vessel.

In accordance with an exemplary embodiment of the present invention, a method is presented for replacing a diseased section of a blood vessel with an IOLP. The diseased section of the blood vessel is transected and the fixing end of the IOLP is attached to one of the transected end of the blood vessel. The inflatable end of the catheter device is inserted into the fixing end of the IOLP and the anvil balloon is inflated. Inflating the anvil balloon presses the attachment means present on the fixing end into the wall of the blood vessel. Using the same method, a similar IOLP is attached to the other transected end of the blood vessel. The two IOLPs are attached to each other by joining their connecting ends. The lumens of the two joined IOLPs act as a conduit for the passage of blood, thus replacing the diseased section of the blood vessel.

Additional objects and advantages of the invention will become apparent to those skilled in the art upon reference to the detailed description taken in conjunction with the provided figures.

### BRIEF DESCRIPTION OF THE DRAWINGS

The various embodiments of the invention will hereinafter be described in conjunction with the appended drawings provided to illustrate and not to limit the invention, wherein like designations denote like elements, and in which:
FIG. 1 is a representation of an In-Out Lay Prosthesis (IOLP) (100), in accordance with one embodiment of the present invention;
FIG. 2 is a representation of attachment means (204) of IOLP (100), in accordance with one embodiment of the present invention;
FIG. 3 is a representation of connecting means (300) of IOLP (100), in accordance with one embodiment of the present invention;
FIG. 4A is a representation of connecting end (108) before the two similar IOLPs (100) are joined together, in accordance with one embodiment of the present invention;
FIG. 4B is a representation of connecting end (108) after two similar IOLPs (100) are joined together, in accordance with one embodiment of the present invention;
FIG. 5 is a representation of catheter device (500), in accordance with one embodiment of the present invention;
FIG. 6 is a representation of the catheter device (500), in accordance with another embodiment of the present invention;
FIG. 7 is a representation of knurled screw (606) of catheter device (500), in accordance with one embodiment of the present invention;
FIG. 8A is a representation of the position of attachment means (110) while inserting fixing end (106) into a lumen of a transected end of blood vessel (802), in accordance with one embodiment of the present invention;
FIG. 8B is a representation of the position of attachment means (110) after fixing end (106) has been inserted into the lumen of the transected end of blood vessel (802), in accordance with one embodiment of the present invention;
FIG. 9 is a flowchart of a method for replacing an aneurysmal section of the abdominal aorta using IOLP (100), in accordance with one embodiment of the present invention;
FIG. 10 is a flowchart of a method for attaching IOLP (100) inside the abdominal aorta without the use of sutures, wherein the length of the stump of the abdominal aorta is more than 1.5 cm, in accordance with one embodiment of the present invention;
FIG. 11 is a flowchart of a method for attaching IOLP (100) inside the abdominal aorta without the use of sutures, wherein the length of the stump of the abdominal aorta is less than 1.5 cm, in accordance with one embodiment of the present invention;
FIG. 12 is a flowchart of a method for introducing the fixing end of IOLP (100) inside a lumen of a transected end of the abdominal aorta, in accordance with one embodiment of the present invention;
FIG. 13 is an illustration of the completed procedure for replacing an aneurysmal section of abdominal aorta (1300), in accordance with one embodiment of the present invention, whereby two similar IOLPs (100) are in place and have been joined with each other by connecting ends (108);
FIG. 14 is a representation of pipe clip (1400), to be used in connection with the IOLP according to the invention.
FIG. 15 is an illustration of the completed procedure for replacing an aneurysmal section of abdominal aorta (1300), whereby IOLP (100) is applied to abdominal aorta (1300) in conjunction with pipe clip (1400), in accordance with one embodiment of the present invention; and
FIG. 16 is a illustration of IOLP (100) and IOLP (1600) applied to abdominal aorta (1300) in conjunction with pipe clip (1400), wherein IOLP (1600) has two fixing ends (1604) and one connecting end (1606).

### DETAILED DESCRIPTION OF THE INVENTION

The present invention discloses a vascular prosthesis for replacing a diseased section of a blood vessel and a catheter device for fixing the vascular prosthesis inside a blood vessel. In particular, the present invention discloses an In-Out-Lay-Prosthesis (IOLP) for replacing an aneurysmal section of the abdominal aorta. The aneurysmal section of the abdominal aorta is transected and sutureless anastomosis is established between the IOLP and the abdominal aorta at each of the two transected ends. The two IOLPs fixed at both the transected ends of the abdominal aorta are joined with each other, replacing the aneurysmal section of the abdominal aorta.

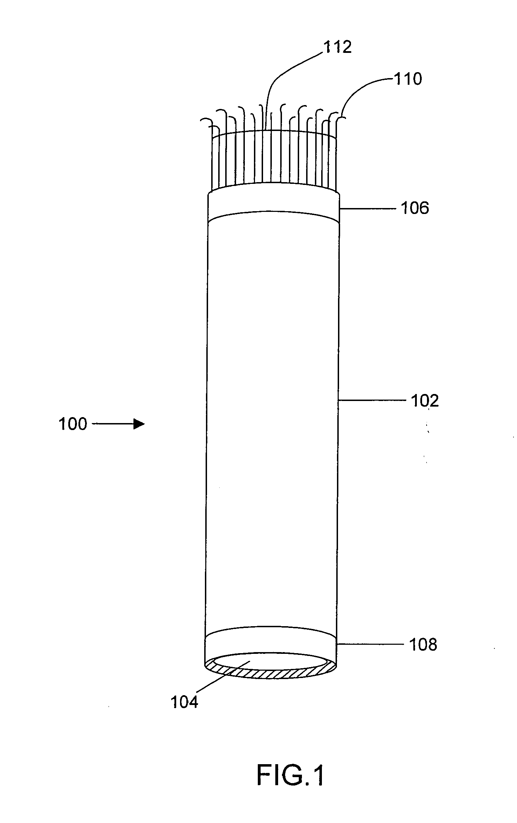

FIG. 1 is a representation of IOLP 100, which is used for replacing the diseased section of a blood vessel, in accordance with one embodiment of the present invention. IOLP 100 has a tubular body member 102, a lumen 104, a fixing end 106, and a connecting end 108. Lumen 104 acts as a conduit for the passage of blood. Attachment means 110 are present on fixing end 106, which help in securing IOLP 100 to the wall of the blood vessel. A membrane 112 is present on fixing end 106. Attachment means 110 are embedded in membrane 112. Membrane 112 provides support to attachment means 110 in order to achieve a secure fixation of IOLP 100 inside the blood vessel.

The length and the diameter of the diseased section of the blood vessel can be determined pre-operatively by using various diagnostic modalities, such as contrast-enhanced computerized tomography. IOLP 100 having the appropriate dimensions can thus be chosen pre-operatively.

In accordance with one embodiment of the present invention, attachment means 110 are of at least two different lengths are attached to fixing end 106 of IOLP 100. This arrangement of attachment means of at least two different lengths helps attachment means 110 to penetrate the wall of the blood vessel at, at least, two different levels. This helps to prevent dislodgement of IOLP 100 inside the blood vessel.

In accordance with one embodiment of the present invention, the thickness of the wall of tubular body member 102 is in a range of 1 mm to 3 mm, the preferred thickness being 2 mm. In accordance with one embodiment of the present invention, tubular body member 102 is made of a biocompatible material. In accordance with one embodiment of the present invention, the biocompatible material is polytetrafluoroethylene (PTFE).

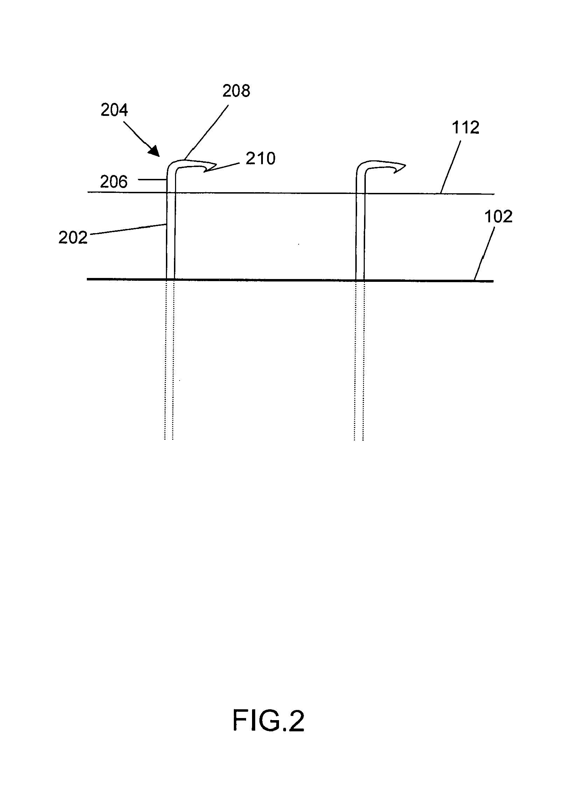

FIG. 2 is a representation of attachment means 110, in accordance with one embodiment of the present invention. Attachment means 110 has a straight part 202 and a hook 204. In accordance with one embodiment of the present invention, straight part 202 is embedded in tubular body member 102 and membrane 112. Hook 204 has an angular part 206 and a penetrating part 208. Penetrating part 208 has a pointed tip 210 that penetrates the wall of the blood vessel and prevents the dislodgement of IOLP 100.

In accordance with one embodiment of the present invention, the length of straight part 202, which is embedded in tubular body member 102, is in a range of 15 mm to 25 mm, the preferred length being 20 mm. In accordance with one embodiment of the present invention, the length of straight part 202 embedded in membrane 112 is in a range of 5 mm to 15 mm, the preferred length being 7.5 mm.

In accordance with one embodiment of the present invention, the length of angular part 206 is in a range of 2 mm to 5 mm, the preferred length being 2.5 mm. In accordance with one embodiment of the present invention, the angle that angulated part 206 makes with penetrating part 208 is in a range of 70° to 90°, the preferred angle being 85°. In accordance with one embodiment of the present invention, the length of penetrating part 208 is in a range of 1-3 mm, the preferred length being 2mm.

In accordance with one embodiment of the present invention, attachment means 110 are made of an elastic biocompatible material. In accordance with one embodiment of the present invention, the elastic biocompatible material is titan.

In accordance with one embodiment of the present invention, the length of membrane 112 is equal to the length of straight part 202 of attachment means 110 embedded in membrane 112. In accordance with one embodiment of the present invention, the thickness of membrane 112 is in a range of 0.1 mm to 1 mm, the preferred thickness being 0.2 mm. In accordance with one embodiment of the present invention, membrane 112 is made of a flexible biocompatible material. In accordance with one embodiment of the present invention, the flexible biocompatible material is a mesh of PTFE.

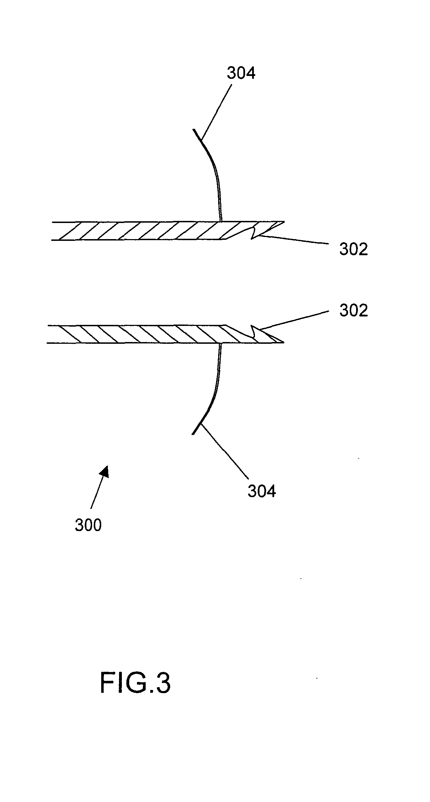

FIG. 3 is a representation of connecting means 300 of IOLP 100, in accordance with one embodiment of the present invention. In accordance with one embodiment of the present invention, connecting means 300 has a circular latch 302 and a cover flap 304. Circular latch 302 attaches with a complementary circular latch on connecting end 108 of another similar IOLP 100. Cover flap 304 covers the joint between two connecting ends 108 of two similar IOLP 100. In accordance with one embodiment of the present invention, circular latch 302 is made of biocompatible material. In accordance with one embodiment of the present invention, the biocompatible material is silicone. In accordance with another embodiment of the present invention, the biocompatible material is PTFE.

In accordance with one embodiment of the present invention, cover flap 304 is made of biocompatible material. In accordance with one embodiment of the present invention, the biocompatible material is PTFE.

In accordance with one embodiment of the present invention, cover flap 304 is fixed over the joint between two connecting ends 108 by using a strand ligature. In accordance with another embodiment of the present invention, cover flap 304 is fixed over the joint between two connecting ends 108 by using a surgical adhesive known in the art such as Bioglue® by Cryolife, Inc.

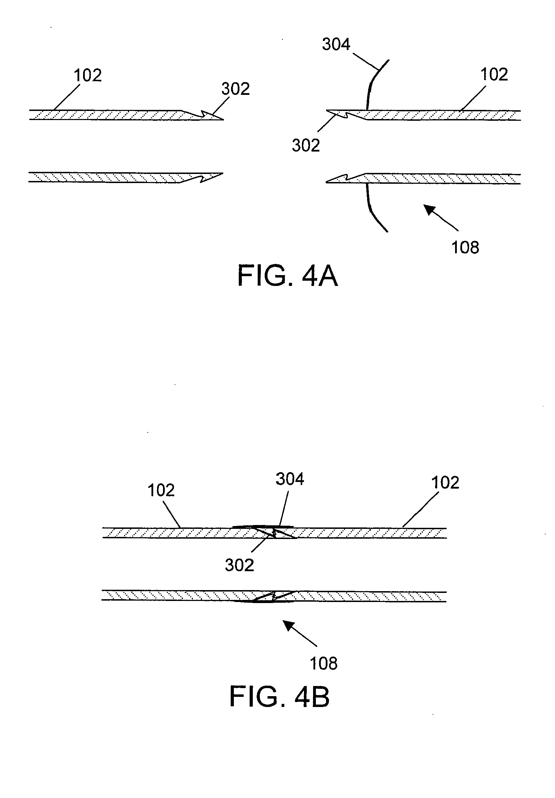

FIG. 4A and FIG. 4B show the use of connecting means 300 for joining connecting end 108 of IOLP 100 with connecting end 108 of another similar IOLP 100, in accordance with one embodiment of the present invention. FIG. 4A is a representation of the connecting ends 108 before joining of two similar IOLP 100. FIG. 4B is a representation of connecting ends 108 after two similar IOLP 100 are joined.

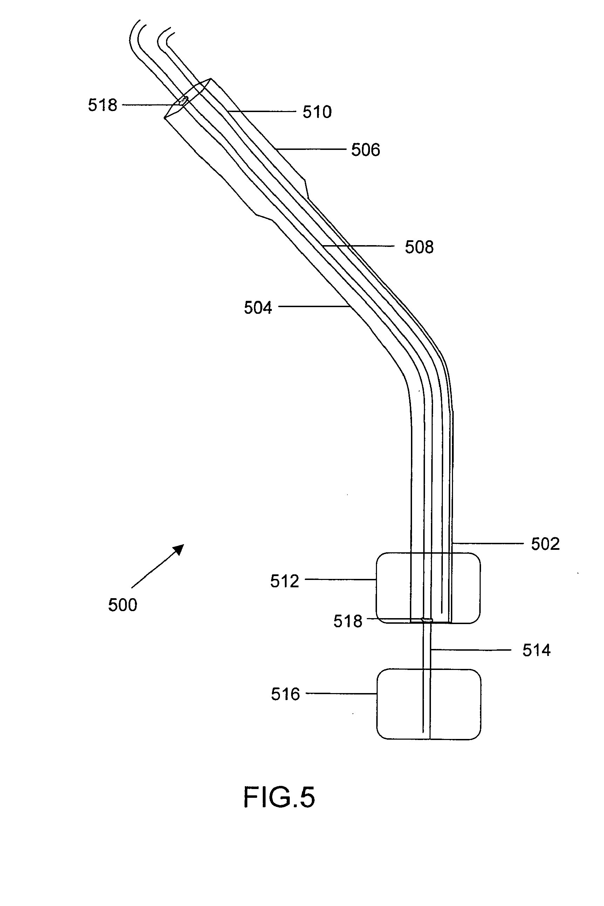

FIG. 5 is a representation of catheter device 500 for fixing IOLP 100 inside a blood vessel, in accordance with one embodiment of the present invention. Catheter device 500 has an inflatable end 502, a body 504, and a handgrip 506.

In accordance with one embodiment of the present invention, body 504 has a curved shape. The curved shape of body 504 helps in using catheter device 500 during open surgery. Handgrip 506 is used to hold and maneuver catheter device 500. Body 504 and handgrip 506 have a channel 508 and a passage 510. In accordance with one embodiment of the present invention, channel 508 is used for inflating anvil balloon 512. Passage 510 is used for introducing an occluding catheter 514 having occluding balloon 516 at its tip. In accordance with one embodiment of the present invention, the collapsed occluding balloon 516 can be withdrawn from catheter device 500 by pulling occluding catheter 514 out of passage 510.

In accordance with one embodiment of the present invention, occluding balloon 516 and anvil balloon 510 are made of flexible biocompatible material. In one embodiment of the present invention, the flexible biocompatible material is silicone.

In accordance with one embodiment of the present invention, the length of catheter device 500 is in a range of 280 mm to 550 mm, the preferred length being 430 mm. In accordance with one embodiment of the present invention, the diameter of body 504 is in a range of 7mm to 12 mm, the preferred diameter being 9 mm.

In accordance with one embodiment of the present invention, body 504 of catheter device 500 is made of a biocompatible material. In one embodiment of the present invention, the biocompatible material is Poly Vinyl Chloride (PVC).

In accordance with one embodiment of the present invention, handgrip 506 is made of a biocompatible material. In accordance with one embodiment of the present invention, the biocompatible material is PVC.

The curved shape of body 504 of catheter device 500 helps in using catheter device 500 during open surgery. During such a procedure, inflatable end 502 of catheter device 500 is inserted into fixing end 106 of IOLP 100. However, during a laparoscopic procedure for replacing a diseased section of a blood vessel, catheter device 500 has to be inserted into a body cavity through ports.

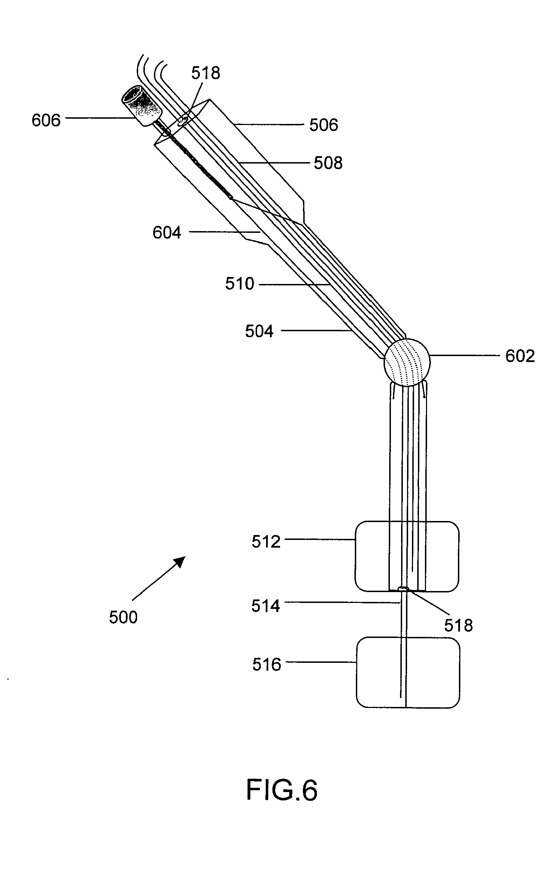

These ports have trocars that are commonly straight. It is difficult to insert curved instruments through these straight trocars. In order to overcome this difficulty, a catheter device that can change from a straight to a curved configuration is required. FIG. 6 is a representation of a variation in catheter device 500, in accordance with one embodiment of the present invention. In this embodiment body 504 of catheter device 500 has a joint 602 that allows body 504 to change from a straight to an angular shape. In accordance with one embodiment of the present invention, joint 602 is a hinge joint. In accordance with one embodiment of the present invention, body 504 and handgrip 506 have two similar longitudinal members 604 that help in controlling the movement of joint 602.

In accordance with one embodiment of the present invention, each longitudinal member 604 is made of steel wires. In accordance with one embodiment of the present invention, body 504 and handgrip 506 have channels for the passage for two similar longitudinal members 604. In accordance with one embodiment of the present invention, handgrip 506 has a knurled screw 606 that is used to control the movement of longitudinal members 604.

In accordance with one embodiment of the present invention, handgrip 506 has an airtight valve 518 at the two ends of passage 510. Valve 518 helps in avoiding air loss due to aeroperitoneum and backflow of blood through passage 510 while occluding catheter 514 is not being used. In accordance with one embodiment of the present invention, valve 518 is made of a biocompatible material. In accordance with one embodiment of the present invention, the biocompatible material is a silicone membrane.

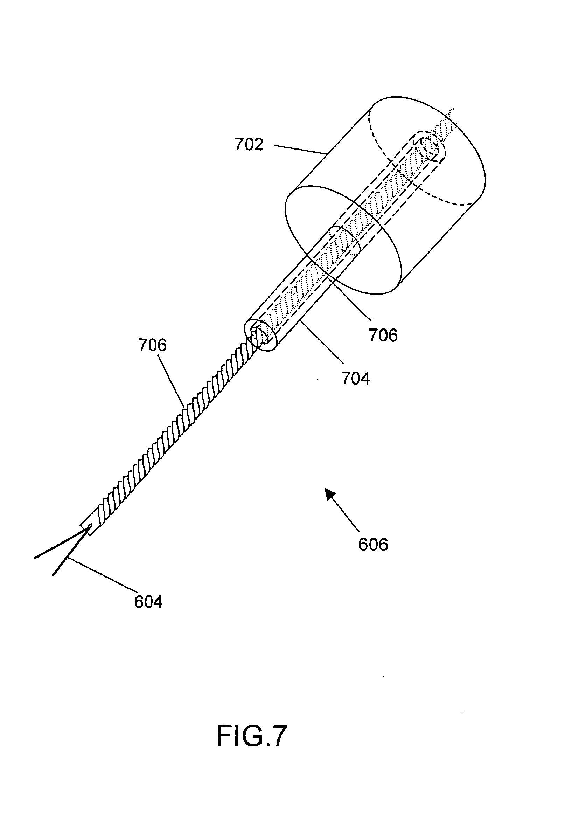

FIG. 7 is a representation of knurled screw 606, in accordance with one embodiment of the present invention. Knurled screw 606 has a knurled knob 702, a sleeve 704 and, a threaded pin 706. Knurled knob 702 is fixed to sleeve 704. Sleeve 704 has a threaded channel 708 for the passage of threaded pin 706.

In accordance with one embodiment of the present invention, one end of longitudinal member 604 is attached to threaded pin 706 and the other end is fixed to the part of body 504 between joint 602 and inflatable end 502. Upon turning knurled knob 702, threaded pin 706 moves longitudinally in threaded channel 708. This causes the tension in longitudinal member 604 to increase. The increased tension in longitudinal members 604 helps to fix joint 602 in position. During the turning of knurled knob 702, torque applied to knurled knob is transmitted to threaded pin 706 as a longitudinal force that pulls longitudinal members 604. The surface of handgrip 506 abuts upon sleeve 704 during turning and acts as a counter bearing.

In accordance with one embodiment of the present invention, the diameter of knurled knob 702 is in a range of 10 mm to 14 mm, the preferred diameter being 12 mm. In accordance with one embodiment of the present invention, the diameter of threaded channel 708 is in a range of 2 mm to 4 mm, the preferred diameter being 3 mm. In accordance with one embodiment of the present invention, the diameter of threaded pin 706 is in a range of 1.5 mm to 3.5 mm, the preferred diameter being 2.5 mm.

In accordance with one embodiment of the present invention, knurled knob 702 is made of a metal alloy. In accordance with one embodiment of the present invention, the metal alloy is steel. In accordance with one embodiment of the present invention, sleeve 704 is made of a metal alloy. In accordance with one embodiment of the present invention, the metal alloy is steel. In accordance with one embodiment of the present invention, threaded pin 706 is made of a metal alloy. In accordance with one embodiment of the present invention, the metal alloy is steel.

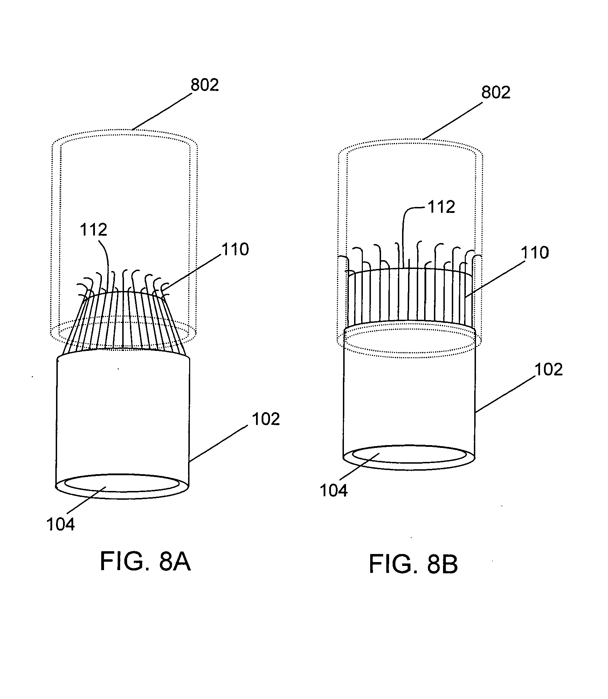

FIG. 8A and FIG. 8B represent the use of IOLP 100 for replacing a diseased section of a blood vessel 802, in accordance with one embodiment of the present invention. Fixing end 106 of IOLP 100 is introduced in the lumen of blood vessel 802. While introducing fixing end 106 of IOLP 100, attachment means 110 are folded towards lumen 104 of IOLP 100.

FIG. 8A is a representation of the position of attachment means 110 while inserting fixing end 106 into the lumen of blood vessel 802. After introducing fixing end 106 into the lumen of blood vessel 802, attachment means 110 are straightened. FIG.8B is a representation of the position of attachment means 110 after fixing end 106 has been inserted into the lumen of blood vessel 802.

In accordance with one embodiment of the present invention, attachment means 110 are folded towards lumen 104 of IOLP 100 by binding them with a suture. In accordance with one embodiment of the present invention, the suture is an absorbable suture known in the art, such as Vicryl rapid® by Ethicon, Johnson and Johnson.

Having disclosed IOLP 100, catheter device 500 for fixing IOLP 100 inside a blood vessel, a method for replacing a diseased section of a blood vessel by using these is disclosed hereinafter. As an example of replacing a diseased section of a blood vessel, a procedure for replacing a section of the abdominal aorta is described. The diseased section may be an aneurysm of the abdominal aorta or an obliterated section of the abdominal aorta. It will be apparent to one skilled in the art that the same procedure can be applied to replace a diseased section of any other blood vessel.

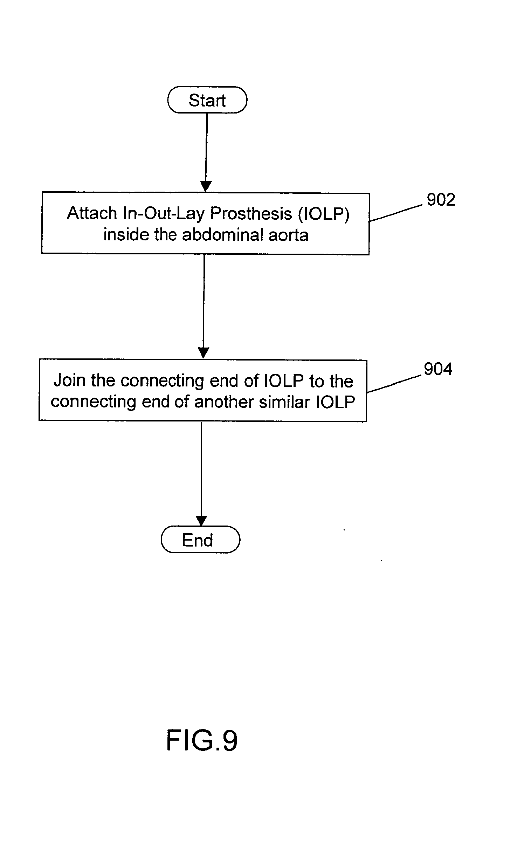

FIG. 9 is a flowchart of a method for replacing an aneurysmal section of the abdominal aorta, in accordance with one embodiment of the present invention. Access to the abdominal aorta is gained by using a trans-peritoneal or a retro-peritoneal access. At step 902, IOLP 100 is attached inside the abdominal aorta. At step 904, connecting end 108 of IOLP 100 is joined with connecting end 108 of another similar IOLP 100. Joining connecting end 108 of one IOLP 100 to connecting end 108 of another similar IOLP 100 is achieved by interlocking circular latch 302 on connecting end 108 of one IOLP 100 with a complementary circular latch 302 on connecting end 108 of another similar IOLP 100.

In accordance with one embodiment of the present invention, cover flap 304 is then secured over the joint between the two connecting ends 108 by applying a ligature. The ligature can be applied by using a non-absorbable suture material known in the art, such as Ethilon® by Ethicon, Johnson and Johnson.

In accordance with one embodiment of the present invention, cover flap 304 secured over the joint between the two connecting ends 108 by applying a surgical adhesive known in the art such as Bioglue® by Cryolife, Inc.

The step of attaching IOLP 100 inside the abdominal aorta is described in detail hereinafter. Step 902 of attaching IOLP 100 inside the abdominal aorta can be accomplished by different methods depending upon the length of the stump of the abdominal aorta. The length of the stump of the abdominal aorta is the length of the abdominal aorta between the transected end of the abdominal aorta and the origin of any of the main branches of the abdominal aorta, such as renal arteries.

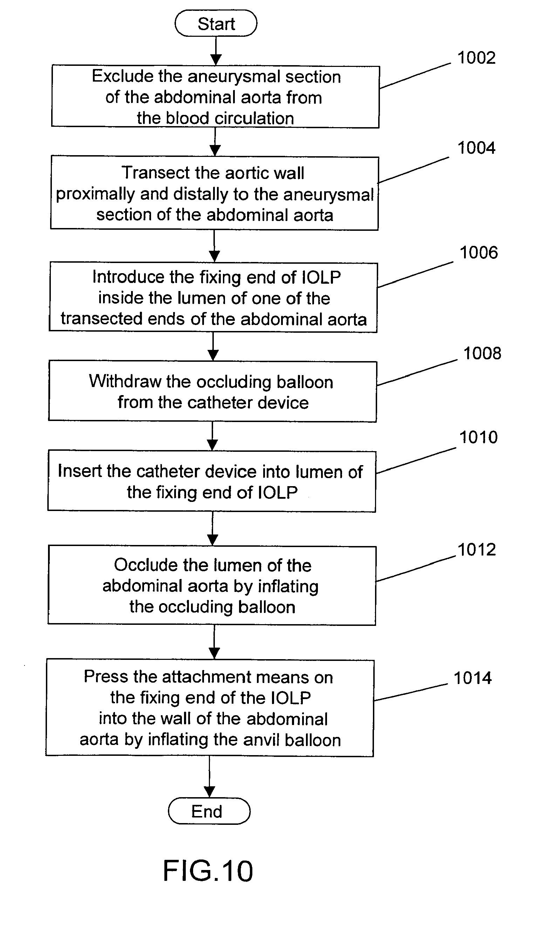

FIG. 10 is a flowchart of a method for attaching IOLP 100 inside the abdominal aorta without the use of sutures, wherein the length of the stump of the abdominal aorta is more than 1.5 cm. At step 1002, the aneurysm is excluded from the blood circulation by applying vascular clamps proximally and distally to the aneurysmal section of the abdominal aorta. Vascular clamps such as Fogarty aortic clamp may be used for this purpose. At step 1004, the wall of the abdominal aorta is transected proximally and distally to the aneurysmal section of the abdominal aorta. At step 1006, fixing end 106 of IOLP 100 is introduced into the lumen of one of the transected ends of the abdominal aorta. At step 1008, occluding catheter 514 is withdrawn from catheter device 500 by pulling it out of passage 510. At step 1010, inflatable end 502 of catheter device 500 is inserted into the lumen of fixing end 106 of IOLP 100. At step 1012, attachment means 110 are pressed into the wall of the abdominal aorta by inflating anvil balloon 512. In accordance with one embodiment of the present invention, anvil balloon 512 is inflated by injecting normal saline into channel 508. Upon pressing the attachment means into the wall of the abdominal aorta, penetrating part 208 of attachment means 110 pierces the wall of the abdominal aorta. In this way fixation of IOLP 100 is achieved inside the abdominal aorta without the use of sutures.

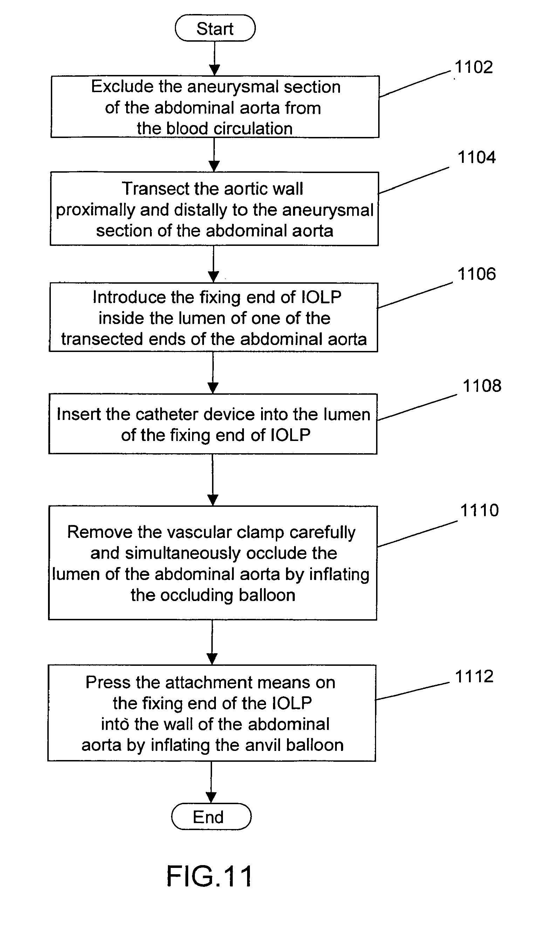

FIG. 11 is a flowchart of a method for attaching IOLP 100 inside the abdominal aorta without the use of sutures, wherein the length of the stump of the abdominal aorta is less than 1.5 cm. At step 1102, the aneurysm is excluded from blood circulation by applying vascular clamps proximally and distally to the aneurysmal section of the abdominal aorta. These vascular clamps are known in the art such as Fogarty aortic clamp. At step 1104, the wall of the abdominal aorta is transected proximally and distally to the aneurysmal section of the abdominal aorta. At step 1106, fixing end 106 of IOLP 100 is introduced into the lumen of one of the transected ends of the abdominal aorta. At step 1108, inflatable end 502 of catheter device 500 is inserted into the lumen of fixing end 106 of IOLP 100. At step 1110, vascular clamp applied proximal to the diseased section of the blood vessel is carefully removed while simultaneously inserting occluding catheter 514 and inflating occluding balloon 516 in the lumen of the abdominal aorta. Removing the vascular clamp and occluding the lumen of the abdominal aorta with occluding balloon 516 provides space for inflating anvil balloon 512. At step 1112, attachment means 110 are pressed into the wall of the abdominal aorta by inflating anvil balloon 512. In accordance with one embodiment of the present invention, anvil balloon 512 is inflated by injecting normal saline into channel 508. Upon pressing the attachment means into the wall of the abdominal aorta, penetrating part 208 of attachment means 110 pierces the wall of the abdominal aorta. In this way fixation of IOLP 100 is achieved inside the abdominal aorta without the use of sutures.

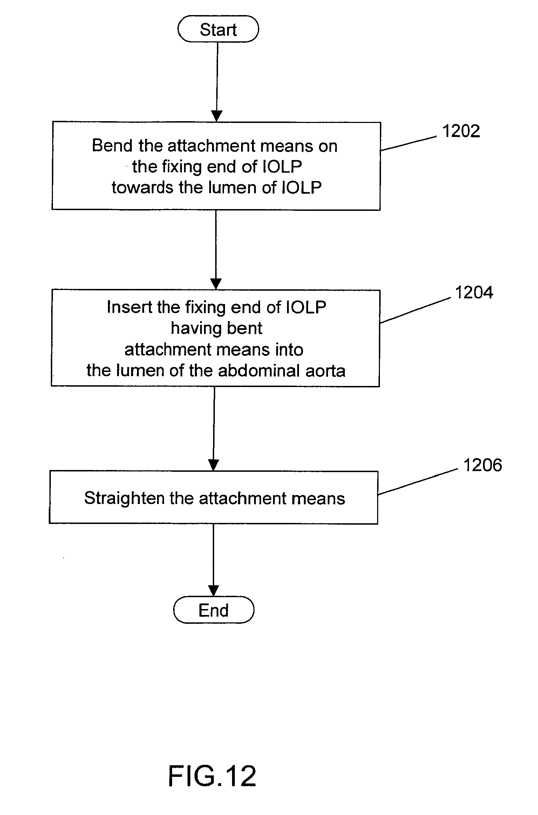

FIG. 12 is a flowchart of a method for introducing fixing end 106 of IOLP 100 into the lumen of the transected end of the abdominal aorta. At step 1202, attachment means 110 are bent towards lumen 104 of tubular body member 102. Attachment means 110 are bent by binding them with a suture. In accordance with one embodiment of the present invention, the suture is an absorbable suture known in the art, such as Vicryl Rapid® by Ethicon, Johnson and Johnson. At step 1204, fixing end 106 is inserted into the lumen of the abdominal aorta. At step 1206, attachment means 110 are straightened by removing the suture.

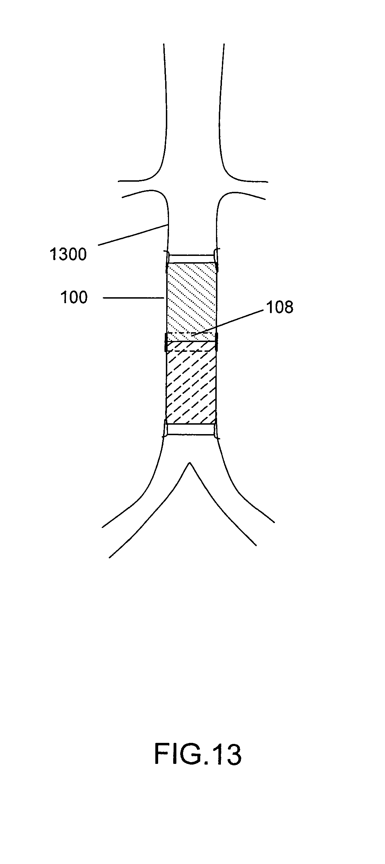

FIG. 13 is an illustration of the completed procedure for replacing the aneurysmal section of abdominal aorta 1300, in accordance with one embodiment of the present invention, whereby two similar IOLP 100 are in place and have been joined with each other by their connecting end 108.

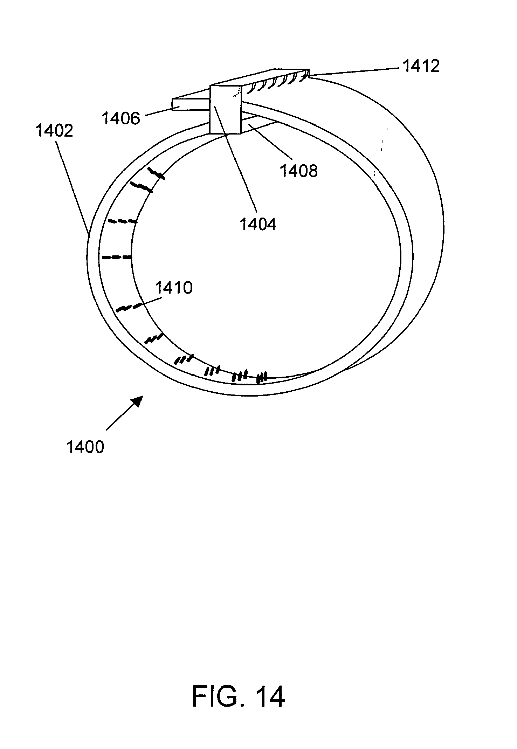

In accordance with one embodiment of the present invention, IOLP 100 is used in conjunction with a pipe clip as described hereinafter. Fig 14 is a representation of such a pipe clip 1400.

Pipe clip 1400 has a body 1402 and a connector 1404 on one end of the body. Body 1402 has a free end 1406 and a fixed end 1408. Connector 1404 is attached on fixed end 1408 of body 1402. Free end 1406 of body 1402 is fixed to connector 1404. Connector 1404 has projections 1412 that fix free end 1406 of body 1402 to connector 1404. Body 1402 also has attachment means 1410 that penetrate the outer wall of the blood vessel and the wall of tubular body member 102 of IOLP 100. Fixation of pipe clip 1400 with the help of attachment means 1410 leads to the formation of a leak proof anastomosis.

Such type of the pipe clip basically comprises a body member having a fixed end and a free end, the body member comprising a plurality of wire members, wherein the plurality of wire members form a wire lattice, and a plurality of attachment means, wherein the plurality of attachment means are attached to the plurality of wire members. Furthermore, the pipe clip comprises a connector which is attached to the free end of the body member.

Herein, preferably at least one of the plurality of attachment means comprises a straight part which is attached to one of the plurality of wire members, and a tip which has a pointed end, the pointed end helping to fix the pipe clip over the site of the anastomosis.

Further preferably, the connector comprises a plurality of projections, the plurality of projections helping to fix the free end of the body member to the connector.

In a preferred embodiment, the pipe clip further comprises traction means which help to apply force to close the pipe clip at the site of the anastomosis. At least one of the traction means is preferably attached to the free end of the body member, or to the connector, respectively.

Further preferably, the body member is made of a compressible biocompatible material.

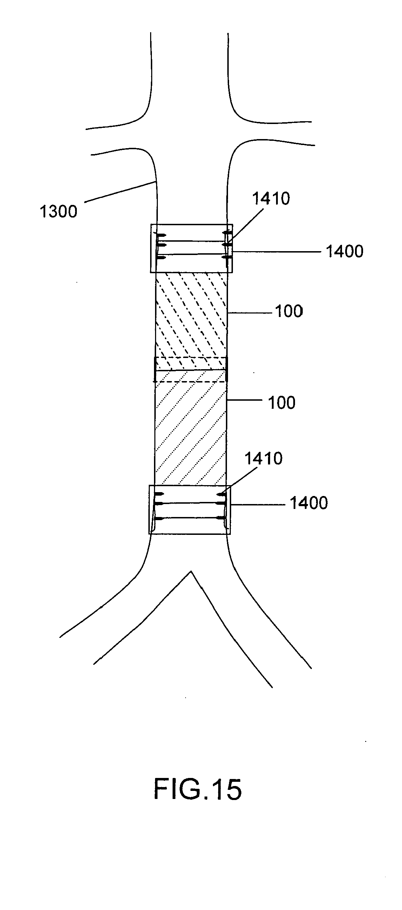

FIG. 15 is an illustration of two similar IOLP 100 applied to abdominal aorta 1300 in conjunction with pipe clip 1400, in accordance with one embodiment of the present invention. Pipe clip 1400 has been applied around the anastomosis between IOLP 100 and abdominal aorta 1300. Fixation of pipe clip 1400 with help of attachment means 1410 leads to the formation of a leak proof anastomosis.

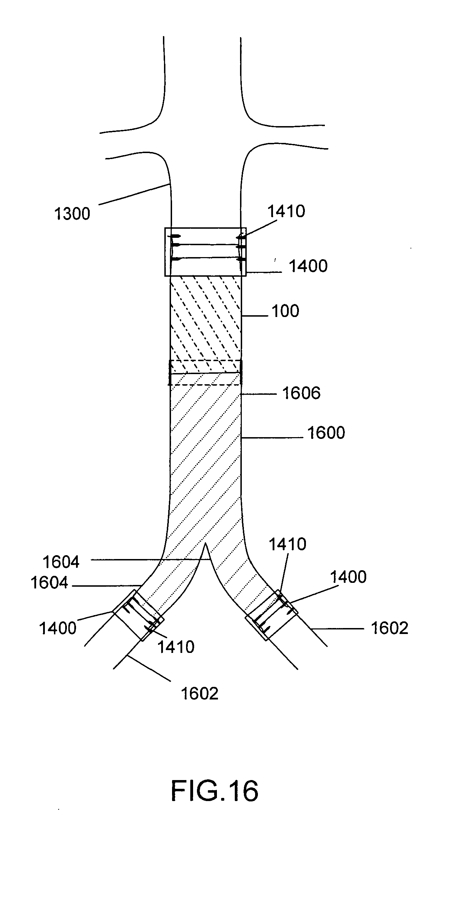

FIG. 16 is an illustration of use of IOLP 100 and a variant of IOLP, IOLP 1600, and pipe clip applied to abdominal aorta 1300 and common iliac arteries 1602, in accordance with one embodiment of the present invention. IOLP 1600 has two fixing ends 1604 and one connecting end 1606, in accordance with another embodiment of the present invention. Two fixing ends 1604 of IOLP 1600 are used to form anastomosis with the two common iliac arteries 1602. Pipe clip 1400 has been applied around the anastomosis between IOLP 100 and abdominal aorta 1300 as well as the anastomosis between IOLP 1600 and the two common iliac arteries 1602. Fixation of pipe clip 1400 with help of attachment means 1410 leads to the formation of a leak proof anastomosis.

While the various embodiments of the invention have been illustrated and described, it will be clear that the invention is not limited to these embodiments only. Numerous modifications, changes, variations, substitutions, and equivalents will be apparent to those skilled in the art without departing from the spirit and scope of the invention as described in the claims.

## Claims

1. An in-out-lay prosthesis (100) suitable for replacing a diseased section of a blood vessel, the in-out-lay prosthesis comprising:
a. a tubular body member (102), the tubular body member acting as a conduit for the passage of blood, the tubular body member comprising:
i. at least one fixing end (106) for fixing the in-out-lay prosthesis to the wall of the blood vessel;
ii. at least one connecting end (108) for joining one in-out-lay prosthesis with another similar in-out-lay prosthesis;
b. a plurality of attachment means (110) on the fixing end of the tubular body member, the attachment means helping in fixing the in-out-lay prosthesis to the wall of the blood vessel;
c. a membrane (112) on the fixing end of the tubular body member, the membrane helping in supporting the plurality of the attachment means; and
d. a connecting means (300) on the connecting end of the tubular body member, the connecting means helping in joining the in-out-lay prosthesis to another similar in-out-lay prosthesis.

2. The in-out-lay prosthesis of claim 1, **characterized in that** the membrane is made of a flexible biocompatible material.

3. The in-out-lay prosthesis of claim 1 or 2, **characterized in that** at least one of the plurality of the attachment means comprises;
a. a straight part (202), the straight part fixing the attachment means to the tubular body member, the straight part being embedded in the membrane; and
b. a hook (204), the hook securing the in-out-lay prosthesis inside the blood vessel, the hook projecting from the membrane.

4. The in-out-lay prosthesis of one of the preceding claims, **characterized in that** at least one of the plurality of the attachment means is made of an elastic biocompatible material.

5. The in-out-lay prosthesis of one of the preceding claims, **characterized in that** the connecting means comprises:
a. a circular latch (302), the circular latch helping in joining the connecting means of one in-out-lay prosthesis with the connecting means of another similar in-out-lay prosthesis; and
b. a cover flap (304), the cover flap helping in making the joint between two similar in-out-lay prosthesis leak proof.

6. A prosthetic system for replacing a diseased section of a blood vessel, the prosthetic system comprising
a in-out-lay prosthesis (100) according to one of the preceding claims and
a catheter device (500) for fixing the in-out-lay prosthesis inside a blood vessel.

7. The prosthetic system of claim 6, **characterized in that** the catheter device (500) comprises:
a. an inflatable end (502), the inflatable end being inserted into the lumen of the blood vessel;
b. a handgrip (506), the handgrip being used to hold the catheter device; and
c. a body (504) joining the inflatable end and the handgrip.

8. The catheter device of claim 7, **characterized in that** the inflatable end comprises:
a. an occluding balloon (516), the occluding balloon attached to an occluding catheter (514), the occluding balloon helping in occluding the lumen of the blood vessel; and
b. an anvil balloon (512), the anvil balloon helping in pressing the attachment means (110) of the in-out-lay prosthesis (100) into the wall of the blood vessel.

9. The catheter device of claim 8, **characterized in that** the body (504) of the catheter device (500) comprises:
a. a passage (508) for introducing the occluding catheter; and
b. a channel (510) for inflating the anvil balloon.

10. The catheter device of one of the claims 7 to 9, **characterized in that** the body (504) has a rigid angular shape.

11. The catheter device of one of the claims 7 to 10, **characterized in that** the body (504) comprises a joint (602) that allows the body (504) to change between a straight and an angular configuration.

12. The catheter device of claim 11, **characterized in that** the body (504) comprises longitudinal members (604) for controlling the movement of the joint.
